# EUROPEAN PATENT APPLICATION

(11) **EP 4 450 964 A2**
(43) Date of publication of application: **23.10.2024**
(21) Application number: 24171302.3
(22) Date of filing: 19.04.2024
(51) Int. Cl.: G01N 27/327, C12Q 1/00

(54) **ELECTROCHEMICAL BIOSENSOR STRIP, METHOD FOR MEASURING BLOOD GLUCOSE CONCENTRATION, AND ELECTROCHEMICAL QUANTITATIVE ANALYSIS SYSTEM**

(30) Priority: 21.04.2023 US 202363497444 P; 28.09.2023 CN 202311271928
(71) Applicant: DelBio, Inc., Taoyuan 320 (TW)
(72) Inventor: TSAI, Tsung-Hsuan, 320 Taoyuan (TW); CHOU, Yi-An, 320 Taoyuan (TW); YIN, Chien-Yu, 320 Taoyuan (TW); SHEN, Bo-Jiun, 320 Taoyuan (TW)
(74) Representative: Nederlandsch Octrooibureau

(57) **Abstract**

The electrochemical biosensor strip includes an electrode layer, a spacer, a reagent layer and a cover layer. The electrode layer is disposed on a substrate, and the electrode layer includes a first electrode, a second electrode and a third electrode. The spacer is disposed on the electrode layer, and the spacer includes a first opening, a second opening and a groove which are not communicated with each other. The groove is recessed inwardly from a side of the spacer. The first and second openings are adjacent to the groove. The first opening, the second opening and the groove respectively correspond to and expose the first electrode, the second electrode and the third electrode. The reagent layer is disposed on the first electrode and the second electrode, and the reagent layer is not disposed on the third electrode exposed from the groove. The cover layer is disposed on the spacer.

## Description

### BACKGROUND

### Field of Invention

The present disclosure relates to an electrochemical biosensor strip, a method for measuring blood glucose concentration, and an electrochemical quantitative analysis system.

### Description of Related Art

With the progress of technology and change of the human habits, the home health care gets more and more attention. The home health care is not only to monitor the real-time status of patients, but also to bring many inspection items from hospital to patients' home. The most common testing items include the blood glucose test, which is an important step to effectively control and treat diabetes.

However, the portable blood glucose meters used recently exist greater error value which is usually criticized by most users; and the most influential factor is the hematocrit (HCT) value of blood samples which is varied by different ages and situations. For example, newborn baby has a higher HCT resulting in underestimated glucose levels when measuring; the patients with dialysis have lower HCT resulting in over-estimated glucose levels when measuring. Hence, many kinds of methods used for testing hematocrit of blood sample are developed.

The methods recently used for testing the hematocrit include fluid-velocity method, spectroscopic method, filtration by membrane, and especially the electrochemical method. The electrochemical method adapts electrochemical sensing strips to test the hematocrit of the testing solution, and then, to compensate the blood glucose with numerical calculation to make the testing result more close to the real value of the user.

However, some electrochemical method still remains some limitations. For example, the conventional strip structure is too complicated to simplify the manufacturing procedure, nor to decrease the manufacturing time. Otherwise, the accuracy of the self-testing results by patients is still not enough because of the error affected by the hematocrit in the blood sample and the method of the blood glucose compensation.

Therefore, how to provide a correction method that effectively eliminates the influence of glucose or interfering substances on blood glucose values and hematocrit ratios to obtain a more accurate hematocrit ratio, thereby accurately correcting blood glucose values has become one of the important issues.

### SUMMARY

One aspect of the present disclosure provides an electrochemical biosensor strip, which adopts a three-electrode concept design and uses a spacer to define chemical reaction areas to reduce variation in reaction areas. The electrochemical biosensor strip includes an electrode layer, a spacer, a reagent layer and a cover layer. The electrode layer is disposed on a substrate, and the electrode layer includes a first electrode, a second electrode and a third electrode. The spacer is disposed on the electrode layer, and the spacer includes a first opening, a second opening and a groove which are not communicated with each other. The groove is recessed inwardly from a side of the spacer. The first opening and the second opening are adjacent to the groove. The first opening, the second opening and the groove respectively correspond to and expose the first electrode, the second electrode and the third electrode. The reagent layer is disposed on the first electrode and the second electrode respectively exposed from the first opening and the second opening, and the reagent layer is not disposed on the third electrode exposed from the groove. The cover layer is disposed on the spacer.

In some embodiments, the first opening is spaced apart from the second opening by a first distance, and the first distance is ranged from about 0.2 mm to about 10 mm.

In some embodiments, an area of the first opening is ranged from about 0.2 mm2 to about 3.14 mm2.

In some embodiments, an area of the second opening is ranged from about 0.2 mm2 to about 3.14 mm2.

In some embodiments, the first electrode is a counter electrode, the second electrode is a working electrode, and the third electrode is a bare electrode.

In some embodiments, the groove, the second opening and the first opening are sequentially arranged from the side of the spacer to the other opposite side, and the bare electrode, the working electrode and the counter electrode are sequentially exposed.

In some embodiments, the first electrode is composed of a carbon layer.

In some embodiments, the second electrode is composed of a carbon layer.

In some embodiments, the third electrode includes a silver layer and a carbon layer disposed on the silver layer.

In some embodiments, an area of the carbon layer is greater than an area of the silver layer.

In some embodiments, the cover layer has a visible area corresponding to the first opening, the second opening, and the groove.

Another aspect of the present disclosure provides a method for measuring blood glucose concentration, which can effectively eliminate the influence of glucose or interfering substances on blood glucose values and hematocrit ratios, thereby obtaining a more accurate hematocrit ratio, thereby accurately correcting blood glucose values. The method for measuring blood glucose concentration includes the following steps. First, a blood sample is provided into a reaction area of an electrochemical biosensor strip. An AC voltage signal is applied to the reaction area to obtain a hematocrit index. A first DC voltage signal is applied to the reaction area to obtain an initial blood glucose index. A second DC voltage signal is applied to the reaction area to obtain a background interference index. The initial blood glucose index is corrected by the hematocrit index and the background interference index to obtain a blood glucose concentration.

In some embodiments, the step of applying the first DC voltage signal to the reaction area includes sequentially and continuously applying a positive voltage DC signal and a negative voltage DC signal.

In some embodiments, the step of applying the first DC voltage signal to the reaction area includes sequentially and continuously applying a negative voltage DC signal and a positive voltage DC signal.

In some embodiments, the step of applying the AC voltage signal to the reaction area includes applying the AC voltage signal to a counter electrode and a bare electrode of the reaction area.

In some embodiments, the step of applying the first DC voltage signal to the reaction area includes applying the first DC voltage signal to a counter electrode and a work electrode of the reaction area.

In some embodiments, the step of applying the second DC voltage signal to the reaction area includes applying the second DC voltage signal to a counter electrode and a bare electrode of the reaction area.

In some embodiments, the step of applying the AC voltage signal, the step of applying the first DC voltage signal, and the step of applying the second DC voltage signal are performed continuously and sequentially.

In some embodiments, the step of applying the second DC voltage signal, the step of applying the AC voltage signal, and the step of applying the first DC voltage signal are performed continuously and sequentially.

Yet another aspect of the present disclosure provides an electrochemical quantitative analysis system. The electrochemical quantitative analysis system includes the electrochemical biosensor test strip as mentioned above and a measurement device. The measuring device is electrically connected to the electrochemical biosensor test strip.

These and other features, aspects, and advantages of the present disclosure will become better understood with reference to the following description and appended claims.

It is to be understood that both the foregoing general description and the following detailed description are by examples, and are intended to provide further explanation of the disclosure as claimed.

### BRIEF DESCRIPTION OF THE DRAWINGS

The disclosure can be more fully understood by reading the following detailed description of the embodiment, with reference made to the accompanying drawings as follows:
Fig. 1 is a schematic diagram of an electrochemical biosensor strip according to an embodiment of the present disclosure.
Fig. 2 is an explosion diagram of an electrochemical biosensor strip according to an embodiment of the present disclosure.
Fig. 3 is a schematic diagram of a detailed electrode layer according to an embodiment of the present disclosure.
Fig. 4 is a flow chart of a method for measuring blood glucose concentration according to an embodiment of the present disclosure.
Fig. 5 is a detection voltage waveform diagram using the method for measuring blood glucose concentration according to some embodiments of the present disclosure.
FIG. 6 is a detection voltage waveform diagram using the method for measuring blood glucose concentration according to some embodiments of the present disclosure.
Fig. 7 is a diagram showing the relationship between admittance and hematocrit ratio under different blood glucose concentration values.
Fig. 8 is a diagram showing the relationship between current and voltage under different blood glucose values.
Fig. 9 is a diagram showing the relationship between current and voltage under different uric acid concentrations.

### DETAILED DESCRIPTION

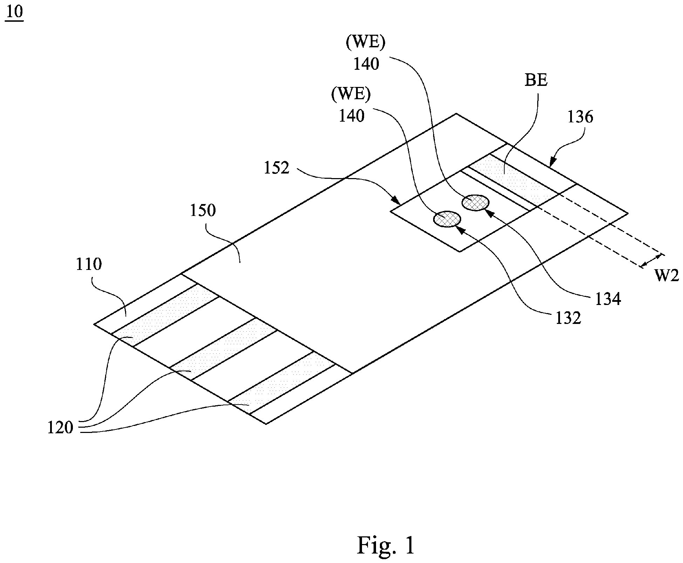

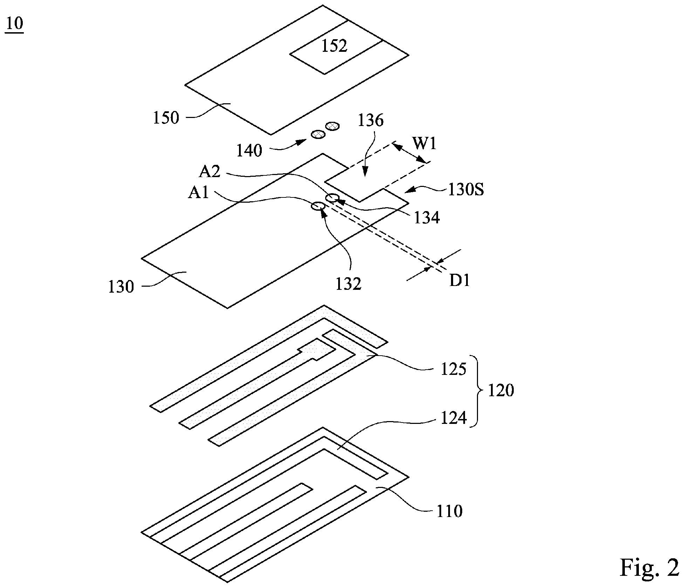

Figs. 1 and 2 are respectively schematic diagrams of the electrochemical biosensor strip 10 after assembly and before assembly according to the embodiment of the present disclosure. Please refer to Fig. 1 and Fig. 2 at the same time. The electrochemical biosensor strip 10 includes a substrate 110, an electrode layer 120, a spacer 130, a reagent layer 140 and a cover layer 150. The electrode layer 120 is disposed on the substrate 110. In some embodiments, the substrate 110 may be an electrically insulating substrate, which may be, for example, but not limited to polyvinyl chloride (PVC), polyethylene (PE), polystyrene (PS), polypropylene (PP), polyether, polyester, polycarbonate (PC), polyethylene terephthalate (PET), polyethylene terephthalate (PETE), silicon dioxide, or aluminum oxide.

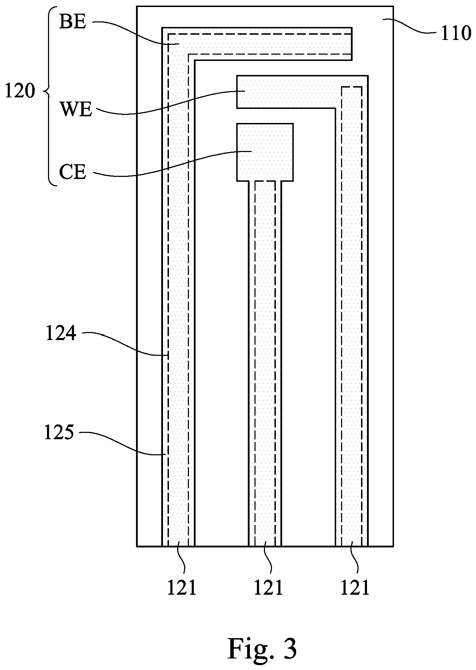

Fig. 3 shows a schematic diagram of the electrode layer 120 on the substrate 110 according to the embodiment of the present disclosure. Please refer to Fig. 2 and Fig. 3 at the same time. The electrode layer 120 includes a first electrode CE, a second electrode WE, and a third electrode BE. More specifically, the first electrode CE is used as a counter electrode for the electrochemical reaction. The second electrode WE is used as a working electrode for glucose reaction current. The third electrode BE is used as a compensation electrode (also called a bare electrode) for interference signals. The first electrode CE, the second electrode WE and the third electrode BE may be, for example, but not limited to, carbon, metal, alloy and/or other conductive materials, in which the metal material may be, for example, gold, silver, palladium, platinum, nickel, copper, molybdenum, cobalt, chromium, zinc, tin, lead or titanium. In addition, the relative positions, shapes and sizes of the first electrode CE, the second electrode WE, and the third electrode BE are not limited by the present disclosure and may be determined based on the actual relative relationship with the electrochemical cells and the direction electrons flow. In some embodiments, the first electrode CE is composed of a carbon layer 125. In some embodiments, the second electrode WE is composed of a carbon layer 125. In some embodiments, the third electrode BE includes a silver layer 124 and a carbon layer 125 disposed on the silver layer 124. In an embodiment where the third electrode BE includes the silver layer 124 and the carbon layer 125, an area of the carbon layer 125 is greater than an area of the silver layer 124. That is to say, the carbon layer 125 completely covers the silver layer 124, and the vertical projection of the carbon layer 125 on the substrate 110 also completely covers the vertical projection of the silver layer 124 on the substrate 110.

It can be understood that the electrode layer 120 also includes three wires 121 extending from the first electrode CE, the second electrode WE, and the third electrode BE respectively. It can be understood that these wires 121 do not necessarily have a straight structure, and these wires 121 can be designed into curved or other irregular shapes according to requirements. In some embodiments, these wires 121 include the silver layer 124 and the carbon layer 125 disposed on the silver layer 124, and the area of the carbon layer 125 is greater than the area of the silver layer 124. That is to say, the carbon layer 125 completely covers the silver layer 124, and the vertical projection of the carbon layer 125 on the substrate 110 also completely covers the vertical projection of the silver layer 124 on the substrate 110. In some embodiments, the electrode layer 120 may obtain the desired pattern through various processing techniques, such as screen printing, laser engraving, sputtering, chemical plating, etc., but is not limited thereto.

Please return to Fig. 1 and Fig. 2. The spacer 130 is disposed on the electrode layer 120. More specifically, the spacer 130 includes a first opening 132, a second opening 134 and a groove 136 that are not connected to each other. The groove 136 is recessed inwardly from one side 130s of the spacer 130, and the first opening 132 and the second opening 134 are adjacent to the groove 136. The first opening 132, the second opening 134 and the groove 136 respectively correspond to and expose the first electrode CE, the second electrode WE and the third electrode BE.

In some embodiments, the spacer 130 may be an electrically insulating substrate, which may be, for example, but not limited to, polyvinyl chloride (PVC), polyethylene (PE), polystyrene (PS), polypropylene (PP), polyether, polyester, polycarbonate (PC), polyethylene terephthalate (PET), polyethylene terephthalate (PETE), silicon dioxide, or aluminum oxide.

In some embodiments, the first opening 132 is spaced apart from the second opening 134 by a first distance D1, and the first distance D1 is ranged from about 0.2 mm to about 10 mm. For example, the first distance D1 may be 0.4mm, 0.6mm, 0.8mm, 1.0mm, 1.5mm, 2.0mm, 2.5mm, 3.0mm, 3.5mm, 4.0mm, 4.5mm, 5.0mm, 5.5mm, 6.0mm, 6.5mm, 7.0mm, 7.5mm, 8.0mm, 8.5mm, 9.0mm, or 9.5mm. According to various embodiments, when the first distance D1 is greater than a certain value, such as 10 mm, the required amount of specimen is too large. On the contrary, when the first distance D1 is less than a certain value, such as 0.2mm, the electrochemical reaction will be unstable.

In some embodiments, the area A1 of the first opening 132 is ranged from about 0.2 mm² to about 3.14 mm². For example, the area A1 of the first opening 132 may be 0.4 mm², 0.6 mm², 0.8 mm², 1.0 mm², 1.2 mm², 1.4 mm², 1.6 mm², 1.8 mm², 2.0 mm², 2.2 mm², 2.4 mm², 2.6 mm², 2.8 mm², 3.0 mm², or 3.1 mm². According to various embodiments, when the area A1 of the first opening 132 is greater than a certain value, such as 3.14mm², the required amount of specimen is too large. On the contrary, when the area A1 of the first opening 132 is less than a certain value, such as 0.2mm², it will cause inaccuracy in the electrochemical reaction. It can be understood that the size of the area A1 of the first opening 132 will determine the reaction area of the corresponding first electrode CE. It should be noted that the size of the first opening 132 is smaller than the size of the first electrode CE. The "size" mentioned here refer to the dimensions composed of the length and width of the component in the top view.

In some embodiments, the area A2 of the second opening 134 is ranged from about 0.2 mm² to about 3.14 mm². For example, the area A2 of the second opening 134 may be 0.4 mm², 0.6 mm², 0.8 mm², 1.0 mm², 1.2 mm², 1.4 mm², 1.6 mm², 1.8 mm², 2.0 mm², 2.2 mm², 2.4 mm², 2.6 mm², 2.8 mm², 3.0 mm², or 3.1 mm². According to various embodiments, when the area A2 of the second opening 134 is greater than a certain value, such as 3.14mm², the required amount of specimen is too large. On the contrary, when the area A2 of the second opening 134 is less than a certain value, such as 0.2mm², it will cause inaccuracy in the electrochemical reaction. It can be understood that the size of the area A2 of the second opening 134 will determine the reaction area of the corresponding second electrode WE. It should be noted that the size of the second opening 134 is smaller than the size of the second electrode WE. The "size" mentioned here refer to the dimensions composed of the length and width of the component in the top view. It can be understood that the area A1 of the first opening may be the same as the area A2 of the second opening 134, or may be different from the area A2 of the second opening 134.

In some embodiments, the groove 136 has a width W1 of about 0.3 mm to about 0.7 mm on the side 130s of the spacer 130. For example, the width W1 of the groove 136 may be 0.4mm, 0.5mm, or 0.6mm. In addition, the relative positions and shapes of the first opening 132, the second opening 134 and the groove 136 are not limited by the present disclosure.

In some embodiments, the third electrode BE exposed from the groove 136 has a line width W2, and the line width W2 is about 0.1 mm to 2.0 mm. For example, the line width W2 may be 0.4mm, 0.6mm, or 0.8mm. According to various embodiments, when the line width W2 is greater than a certain value, such as 2.0mm, the production cost will increase. On the contrary, when the line width W2 is less than a certain value, such as 0.1mm, it will cause inaccuracy in the electrochemical reaction.

Referring to Fig. 1 and Fig. 2, the reagent layer 140 is disposed on the first electrode CE and the second electrode WE exposed from the first opening 132 and the second opening 134 respectively, and the reagent layer 140 is not disposed on the third electrode BE exposed from the groove 136.

In some embodiments, the reagent layer 140 includes at least one electron transfer substance. The electron transfer substance mentioned here can be tetrathiafulvalene, tetracyanoquinodimethan, meldola blue, potassium ferrocyanide, ferrocene or ferrocenedicarboxylic acid, and is not for limitation. Otherwise, the reagent layer 140 used in the present invention also includes enzyme able to react with the substance to be tested, polymers or stabilizer.

Referring to Fig. 1 and Fig. 2, the cover layer 150 disposed on the spacer 130. In some embodiments, the cover layer 150 has a visible area 152 corresponding to the first opening 132, the second opening 134, and the groove 136.

In some embodiments, the cover layer 150 may be an electrically insulating substrate, which may be, for example, but not limited to, polyvinyl chloride (PVC), polyethylene (PE), polystyrene (PS), polypropylene (PP), polyether, polyester, polycarbonate (PC), polyethylene terephthalate (PET), polyethylene terephthalate (PETE), silicon dioxide, or aluminum oxide.

In some embodiments, outside the predetermined visible area 152, the required colors and patterns may be printed by coating, printing, or any other suitable technology. However, the structures above and their relative positions are not restrictive. In other embodiments, the structures of the electrochemical biosensor strip 10 may also change the order of arrangement, or may include other structures between or outside these structures, which the present disclosure is not limited to.

In some embodiments of the present disclosure, the electrochemical biosensor strip 10 adopts a three-electrode concept design and uses the first opening 132 and the second opening 134 of the spacer 130 to define chemical reaction areas to reduce variation in reaction areas.

Another aspect of the present disclosure further provides an electrochemical quantitative analysis system. The electrochemical quantitative analysis system includes the electrochemical biosensor strip 10 as mentioned above (as shown in Figs. 1 and 2) and a measuring device (not shown) electrically connected to the electrochemical biosensor strip 10. In some embodiments, the measuring device may be a detector of the electrochemical biosensor strip 10, such as a blood glucose meter and the like.

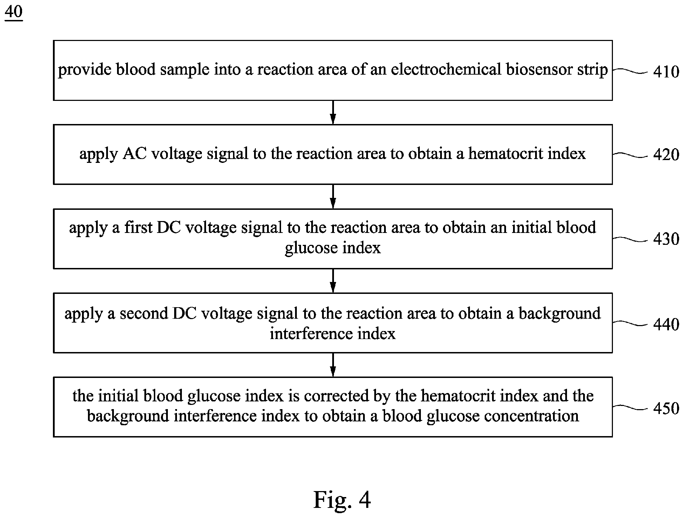

Fig. 4 is a flow chart of a method 40 for measuring blood glucose concentration according to an embodiment of the present disclosure. The method 40 for measuring blood glucose concentration includes at least step 410, step 420, step 430, step 440, and step 450, as shown in Fig. 4. It should be understood that for additional embodiments of the method 40 of measuring blood glucose concentration, additional steps may be provided before, during, and after the method 40, and some of the steps described below may be replaced or eliminated. Furthermore, in order to make the relevant details of each step of this embodiment clearer, the following will be explained with a measuring device (such as a blood glucose meter), that is, the measuring device may be used to perform the method 40 for measuring blood glucose concentration in this embodiment.

In step 410, providing a blood sample into a reaction area of an electrochemical biosensor strip. In some embodiments, the blood sample may be injected into the electrochemical biosensor strip 10 as described in Figs. 1 and 2 and previously described. In detail, by setting the spacer 130 which containing a groove 136 open to the outside world on the substrate 110, the combination of the spacer 130 and the substrate 11 is able to define a space for accommodating blood sample. Therefore, in step 410, the user may inject the blood sample through the groove 136 of the spacer 130, and the blood sample will be guided from the groove 136 to the second opening 134 and the first opening 132 in sequence. The blood sample would sequentially contact the third electrode BE, the second electrode WE, and the first electrode CE to perform subsequent electrochemical reactions with the first electrode CE, the second electrode WE, and the third electrode BE (collectively referred to as the "reaction zone"). In an alternative embodiment, a commercially available electrochemical biosensor strip may also be used to perform the method 40 of measuring blood glucose concentration of the present disclosure. The more detailed electrochemical technology may be understood by those with ordinary knowledge in the technical field, so no further details will be given here. The following embodiments take whole blood as a liquid sample for example.

In step 420, applying an AC voltage signal to the reaction area to obtain a hematocrit index. To be specific, different blood samples contain different amounts of hematocrit (HCT), and the HCT will cause changes in solution impedance. The impedance difference of the solution can be distinguished by measuring the impedance through AC signal and used to identify the HCT in the blood sample, thereby obtaining the hematocrit index. In some embodiments, the AC voltage signal can be applied to the counter electrode CE and the bare electrode BE (the electrodes without the reagent layer) in the reaction area in step 420. In some embodiments, the AC voltage signal has a frequency ranged from about 1 kHz to about 100 kHz. For example, the frequency of the AC voltage signal may be 10 kHz, 20 kHz, 30 kHz, 40 kHz, 50 kHz, 60 kHz, 70 kHz, 80 kHz, or 90 kHz. In some embodiments, the AC voltage signal has a voltage ranged from about 0.05V to about 0.2V. For example, the voltage of the AC voltage signal may be 0.07 V, 0.1 V, 0.13 V, 0.15 V, or 0.17V. In some embodiments, the AC voltage signal is applied for a time ranged from about 0.5 seconds to about 10 seconds. For example, the application time of the AC voltage signal may be 1 second, 2 seconds, 3 seconds, 4 seconds, 5 seconds, 6 seconds, 7 seconds, 8 seconds, or 9 seconds.

In step 430, applying a first DC voltage signal to the reaction area to obtain an initial blood glucose index. More specifically, after applying the AC voltage signal (step 420), the first DC voltage signal is applied to the reaction region, in which the first DC voltage signal includes a positive voltage DC signal and a negative voltage DC signal. It is worth mentioning that the positive voltage DC signal and the negative voltage DC signal are continuously applied to the reaction area. In one embodiment, the positive voltage DC signal may be applied to the reaction area first, and then the negative voltage DC signal may be applied in step 430. In another embodiment, the negative voltage DC signal may be applied to the reaction area first, and then the positive voltage DC signal may be applied in step 430. In some embodiments, the first DC voltage signal may be applied to the counter electrode CE and the working electrode WE in the reaction area in step 430. After completing step 430, an initial induced current signal may be measured, thereby obtaining the initial blood glucose index. In some embodiments, the application time of the first DC voltage signal ranges from about 0.5 seconds to about 10 seconds. For example, the application time of the first DC voltage signal may be 1 second, 2 seconds, 3 seconds, 4 seconds, 5 seconds, 6 seconds, 7 seconds, 8 seconds, or 9 seconds. In some embodiments, the absolute voltage value of the first DC voltage signal ranges from about 0.5V to about 2.0V, such as, 0.6V, 0.8V or 1.0V.

In step 440, applying a second DC voltage signal to the reaction area to obtain a background interference index. In some embodiments, in step 440, the second DC voltage signal is applied to the counter electrode CE and the bare electrode BE in the reaction area. To be specific, the background current of non-glucose reactions may be obtained using the bare electrode as the background interference index. In some embodiments, the second DC voltage signal may be a positive voltage DC signal. In some embodiments, the application time of the second DC voltage signal ranges from about 0.5 seconds to about 10 seconds. For example, the application time of the second DC voltage signal may be 1 second, 2 seconds, 3 seconds, 4 seconds, 5 seconds, 6 seconds, 7 seconds, 8 seconds, or 9 seconds. In some embodiments, the voltage of the second DC voltage signal is about 0.5V to about 2.0V, such as, 0.6V, 0.8V or 1.0V.

In step 450, correcting the initial blood glucose index by the hematocrit index and the background interference index to obtain a blood glucose concentration.

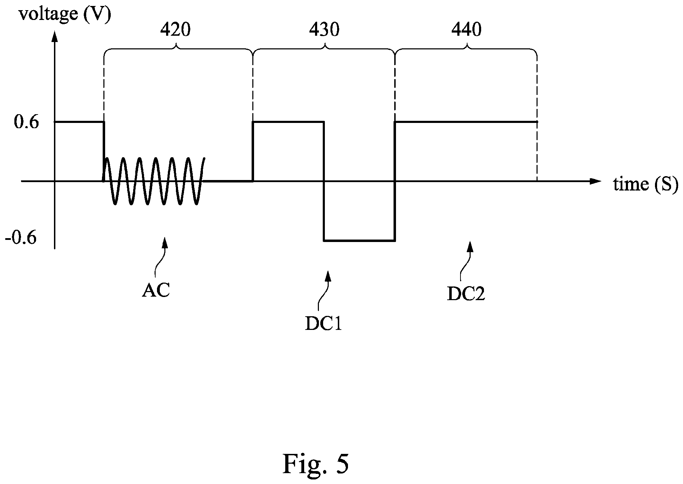

Fig. 5 is a detection voltage waveform diagram using the method for measuring blood glucose concentration according to some embodiments of the present disclosure. In some embodiments, applying the AC voltage signal AC (step 420), applying the first DC voltage signal DC1 (step 430), and applying the second DC voltage signal DC2 (step 440) are performed continuously and sequentially, as shown in Fig. 5. In more detail, a fixed initial voltage may be applied to the reaction area of the electrochemical biosensor strip to confirm whether the blood sample enters the detection area before step 420. Because in a continuous power supply state, the measuring device can immediately detect whether the working electrode and counter electrode in the reaction area are conductive. Once the electrochemical biosensor strip is on-stat, step 420 and subsequent steps 430 and 440 may be continued. In some embodiments, the voltage value of the initial voltage ranges from about 0.5V to about 2.0V, such as, about 0.6V. In addition, the application of the AC voltage may be stopped for a period of time after step 420, where this period of time may be about 0 seconds to 2 seconds, and then converted into the DC voltage signal required in step 430.

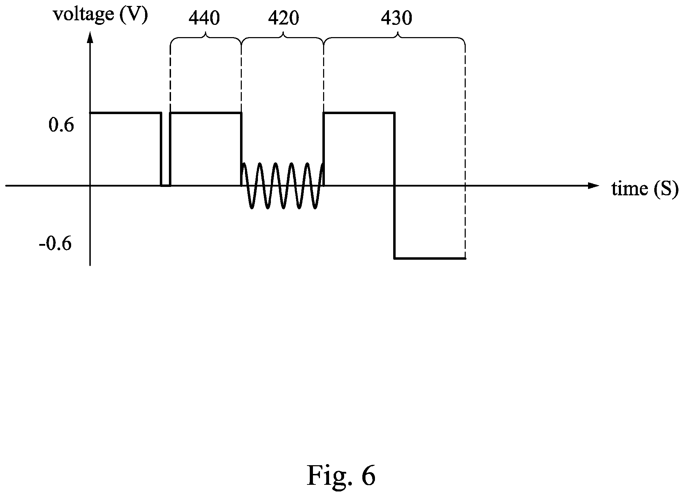

FIG. 6 is a detection voltage waveform diagram using the method for measuring blood glucose concentration according to some embodiments of the present disclosure. The difference from the operation in Fig. 5 is that step 440 may be performed prior to step 420. In some embodiments, applying the second DC voltage signal DC2 (step 440), applying the AC voltage signal AC (step 420), and applying the first DC voltage signal DC1 (step 430) are performed continuously and sequentially, as shown in Fig. 6. In more detail, a fixed initial voltage may be applied to the reaction area of the electrochemical biosensor strip to confirm whether the blood sample enters the detection area before step 440. Because in a continuous power supply state, the measuring device can immediately detect whether the working electrode and counter electrode in the reaction area are conductive. Once the electrochemical biosensor strip is on-stat, step 440 and subsequent steps 420 and 430 may be continued. In some embodiments, the voltage value of the initial voltage is about 0.6V. In some embodiments, after detecting that the blood sample enters the detection area, wait for a period of time (such as, 0 seconds to 5 seconds) before continuing to perform step 440 and subsequent steps 420 and 430.

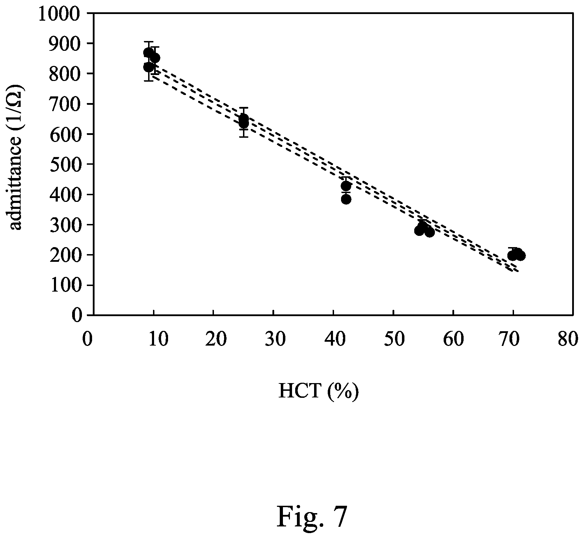

Fig. 7 shows the relationship between the measured admittance and the hematocrit (HCT%) using blood samples with various known blood glucose concentration values(176mg/dL, 273mg/dL, and 370mg/dL). It should be noted that the hematocrit index defined in the present disclosure is admittance, and in power electronics, admittance is defined as the reciprocal of impedance. Each hematocrit may be obtained by electrochemical analysis to obtain the corresponding hematocrit index. It is worth noting that, as can be seen from Fig. 7, the measured hematocrit index will not be affected by the interference of different blood glucose concentration values in the blood sample. In other words, step 420 of the method 40 for measuring blood glucose concentration of the present disclosure may be used to specifically determine the hematocrit index.

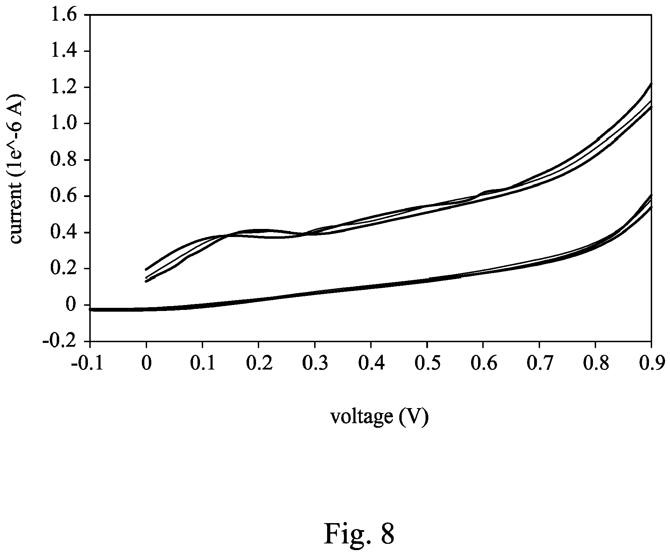

Fig. 8 shows a cyclic voltammetry diagram obtained by using blood samples with various known glucose concentration values (80mg/dL, 190mg/dL, and 480mg/dL). It can be found that the current curves of the three groups of blood samples in this embodiment are very close and have no obvious changes by applying the DC voltage signal to the bare electrode BE of the electrochemical biosensor strip 10 of the present disclosure as shown in Fig. 1. The reason is that the reagent layer 140 is not coated on the bare electrode BE of the electrochemical biosensor strip 10 of the present disclosure, so the glucose does not produce an electrochemical reaction.

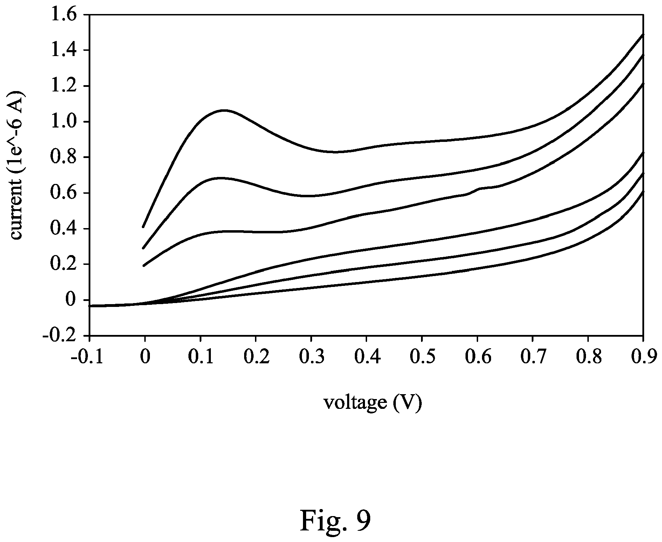

Fig. 9 shows the cyclic voltammetry diagram obtained by using blood samples with various known uric acid concentration values (0mg/dL, 5mg/dL, and 10mg/dL). It should be noted that measuring the blood glucose level of a subject is easily affected by interfering substances, such as uric acid, ascorbic acid, etc. This embodiment takes uric acid as an example to provide the current change of uric acid when the DC voltage signal is applied. As can be seen from Fig. 9, blood samples with different uric acid concentrations will produce obvious oxidation current differences after the DC voltage signal being applied. Therefore, the background interference index of the interfering substances in the specimen may be determined by applying a DC voltage signal to the bare electrode BE of the electrochemical biosensor strip 10 of the present disclosure as shown in Fig. 1.

In summary, the electrochemical biosensor strip of the present disclosure defines the electrochemical reaction area through the spacer, and configures the three electrodes (including the bare electrode, the work electrode, and the counter electrode) on the substrate, so that the three electrodes contact the blood sample and generate an electrochemical reaction, thereby detecting the blood sample. The electrochemical biosensor strip of the present disclosure has a simple structure and only needs to inject a blood sample and follow the simple steps of the method of measuring blood glucose concentration of the present disclosure to obtain the required values, and then the more accurate measurement results may be obtained by correcting between each value. The present disclosure obviously has the effects of simple operation and accurate calibration.

## Claims

1. An electrochemical biosensor strip (10), **characterized by** comprising:
an electrode layer (120) disposed on a substrate (110), the electrode layer comprising a first electrode (CE), a second electrode(WE) and a third electrode (BE);
a spacer (130) disposed on the electrode layer (120), the spacer (130) comprising a first opening (132), a second opening (134), and a groove (136) that are not communicated with each other, the groove (136) recessing inwardly from a side (130s) of the spacer (130), the first opening (132) and the second opening (134) being adjacent to the groove (136), wherein the first opening (132), the second opening (134) and the groove (136) respectively correspond to and expose the first electrode (CE), the second electrode (WE) and the third electrode (BE);
a reagent layer (140) disposed on the first electrode (CE) and the second electrode (WE), wherein the reagent layer (140) is not disposed on the third electrode (BE) exposed from the groove (136); and
a cover layer (150) disposed on the spacer (130).

2. The electrochemical biosensor strip of claim 1, **characterized in that** the first electrode (CE) is a counter electrode, the second electrode (WE) is a working electrode, and the third electrode (BE) is a bare electrode.

3. The electrochemical biosensor strip of claim 2, wherein the groove, the second opening and the first opening are sequentially arranged from the side of the spacer to the other opposite side, and the bare electrode, the working electrode and the counter electrode are sequentially exposed.

4. The electrochemical biosensor strip of any one of claims 1-3, **characterized in that** the third electrode (BE) comprises a silver layer and a carbon layer disposed on the silver layer.

5. The electrochemical biosensor strip of claim 4, wherein an area of the carbon layer is greater than an area of the silver layer.

6. The electrochemical biosensor strip of any one of claims 1-5, wherein the cover layer has a visible area corresponding to the first opening, the second opening, and the groove.

7. A method for measuring blood glucose concentration, **characterized by** comprising steps of:
providing a blood sample into a reaction area of an electrochemical biosensor strip (10);
applying an AC voltage signal to the reaction area to obtain a hematocrit index;
applying a first DC voltage signal to the reaction area to obtain an initial blood glucose index;
applying a second DC voltage signal to the reaction area to obtain a background interference index; and
correcting the initial blood glucose index by the hematocrit index and the background interference index to obtain a blood glucose concentration.

8. The method for measuring blood glucose concentration of claim 7, **characterized in that** the step of applying the first DC voltage signal to the reaction area comprises sequentially and continuously applying a positive voltage DC signal and a negative voltage DC signal.

9. The method for measuring blood glucose concentration of claim 7 or claim 8, **characterized in that** the step of applying the first DC voltage signal to the reaction area comprises sequentially and continuously applying a negative voltage DC signal and a positive voltage DC signal.

10. The method for measuring blood glucose concentration of any one of claims 7-9, **characterized in that** the step of applying the AC voltage signal to the reaction area comprises applying the AC voltage signal to a counter electrode and a bare electrode of the reaction area.

11. The method for measuring blood glucose concentration of any one of claims 7-10, **characterized in that** the step of applying the first DC voltage signal to the reaction area comprises applying the first DC voltage signal to a counter electrode and a work electrode of the reaction area.

12. The method for measuring blood glucose concentration of any one of claim 7-11, **characterized in that** the step of applying the second DC voltage signal to the reaction area comprises applying the second DC voltage signal to a counter electrode and a bare electrode of the reaction area.

13. The method for measuring blood glucose concentration of any one of claim 7-12, **characterized in that** the step of applying the AC voltage signal, the step of applying the first DC voltage signal, and the step of applying the second DC voltage signal are performed continuously and sequentially.

14. The method for measuring blood glucose concentration of any one of claims 7-13, **characterized in that** the step of applying the second DC voltage signal, the step of applying the AC voltage signal, and the step of applying the first DC voltage signal are performed continuously and sequentially.

15. An electrochemical quantitative analysis system, **characterized by** comprising:
the electrochemical biosensor strip of claims 1-6; and
a measuring device electrically connected to the electrochemical biosensor strip.
